# EUROPEAN PATENT SPECIFICATION

(11) **EP 3 957 188 B1**
(45) Date of publication and mention of the grant of the patent: **18.06.2025**
(21) Application number: 20786432.3
(22) Date of filing: 23.09.2020
(51) Int. Cl.: A23L 33/105, A23L 25/00, A61K 36/185, A61P 3/00, C11B 1/10

(54) **METHOD FOR PREPARING SESAME OIL MEAL EXTRACT AND FOOD COMPOSITION COMPRISING SAME AS ACTIVE INGREDIENT FOR PREVENTING OR ALLEVIATING COLITIS**
VERFAHREN ZUR HERSTELLUNG VON SESAMÖLMEHLEXTRAKT UND NAHRUNGSMITTELZUSAMMENSETZUNG DAMIT ALS WIRKSTOFF ZUR VORBEUGUNG ODER LINDERUNG VON COLITIS
PROCÉDÉ DE PRÉPARATION D'EXTRAIT DE FARINE D'HUILE DE SÉSAME ET COMPOSITION ALIMENTAIRE LE COMPRENANT EN TANT QUE PRINCIPE ACTIF POUR LA PRÉVENTION OU LE SOULAGEMENT DE LA COLITE

(30) Priority: 25.06.2020 KR 20200077938
(43) Date of publication of application: 23.02.2022
(73) Proprietor: Queensbucket. Co., Ltd., Iksan-si, Jeonbuk-do 54576 (KR)
(72) Inventor: PARK, Jeongyong, Gochang-gun Jeollabuk-do 56436 (KR); LIM, Yeosook, Seoul 06706 (KR)
(74) Representative: Algemeen Octrooi- en Merkenbureau B.V.
(86) International application number: PCT/KR2020/012832
(87) International publication number: WO 2021/261660

(56) References cited:
- CN-A- 104 814 247
- CN-A- 105 124 132
- CN-A- 107 501 286
- JP-A- 2008 206 479
- KR-A- 20050 015 176
- KR-A- 20180 065 001
- KR-A- 20180 131 222
- US-A1- 2016 136 214
- ELHUSSEIN ELAF ABDELILLAH ALI ET AL: "Cleaner production of micronutrients from sesame seed pressed cake: a comparative study", BIOMASS CONVERSION AND BIOREFINERY, SPRINGER BERLIN HEIDELBERG, BERLIN/HEIDELBERG, vol. 11, no. 4, 12 August 2019 (2019-08-12), pages 1183 - 1196, XP037510510, ISSN: 2190-6815, [retrieved on 20190812], DOI: 10.1007/S13399-019-00483-5
- AKL ENGY M ET AL: "Preparation and characterization of novel antibacterial blended films based on modified carboxymethyl cellulose/phenolic compounds", POLYMER BULLETIN, vol. 78, no. 2, 3 March 2020 (2020-03-03), pages 1061 - 1085, XP037345317, ISSN: 0170-0839, DOI: 10.1007/S00289-020-03148-W
- ESMAEILZADEH KENARI REZA, MOHSENZADEH FATEREH, AMIRI ZEINAB RAFTANI: "Antioxidant activity and total phenolic compounds of Dezful sesame cake extracts obtained by classical and ultrasound‐assisted extraction methods", FOOD SCIENCE & NUTRITION, vol. 2, no. 4, 1 July 2014 (2014-07-01), pages 426 - 435, XP055865376, ISSN: 2048-7177, DOI: 10.1002/fsn3.118

## Description

### [Technical Field]

The present disclosure relates to a method for producing a sesame oil meal extract, a food composition for use in preventing or improving colitis accompanied with weight loss, stool abnormalities, rectal bleeding or a reduction in colon length and Sesame oil meal extract for use in preventing or improving colitis accompanied with weight loss, stool abnormalities, rectal bleeding or a reduction in colon length.

### [Background Art]

Sesame oil meal is a brownish by-product (substance) left after squeezing oil and is called sesame cake. Sesame oil meal has a high crude protein content of 40 to 50%, contains high in calcium and phosphorus in addition to protein, is suitable for the taste of all livestock, and may be stored for a long time.

Sesame oil meal contains a large amount of lignan, which is well known as an antioxidant, as well as nutrients such as protein, dietary fiber, and minerals. In particular, sesaminol glycosides, a kind of lignan contained in sesame oil meal, are known to have various functions such as an anti-oxidation and an inhibition of brain cell damage.

On the other hand, 70% of immunity is determined in the intestine. The bacteria in the intestine break down the food we eat, make it digestible, and also play a role in training the immune system. On the other hand, it may also cause cancer or diarrhea. Therefore, when the intestinal bacteria are well balanced, the intestines and the body are healthy. Recent research also reveals that obesity and various incurable diseases are closely related to these intestinal bacteria and intestinal immunity.

Inflammation is an important immune response that protects against damage or infection by removing harmful elements and repairing cells and tissues. However, excessive acute or chronic inflammation can cause serious disorders such as colitis, arthritis, asthma, colitis, Parkinson's disease, Alzheimer's disease, and sepsis.

Among the inflammatory diseases, colitis is a disease that causes inflammation in the large intestine, and is caused by various causes such as infection, poor blood supply, and autoimmune reactions. Its main symptoms include bloating, lower abdominal pain, diarrhea, etc., and mucus, pus, or blood may be mixed in the stool. Colitis may be largely classified into infectious colitis and non-infectious colitis depending on the cause, and may be classified into acute colitis and chronic colitis depending on the period when a disease is developed. Acute colitis includes amoebic dysentery, bacterial dysentery, and pseudomembranous enteritis caused by salmonella or antibiotics, and chronic colitis includes ulcerative colitis, Crohn's disease, tuberculosis, syphilis, and those caused by X-rays, etc. In addition, colitis includes irritable bowel syndrome (IBS), etc., as well as inflammatory bowel disease (IBD).

In general, for inflammatory bowel disease, biological agents such as anti-TNF agents are used by patients who do not have an effect on steroids and immunosuppressants. According to the research, it is known that 20 to 40% of patients who have been treated with anti-TNF agents lose an effect within one year due to resistance. In addition, there are side effects that increase the risk of opportunistic infection, tuberculosis, latent tuberculosis activation, etc., because the immune suppression occurs throughout the body. Due to these problems, there is a need to develop a health functional food that has no side effects and may be taken for a long time.

In addition, a functional raw material that have been developed up to now as raw material for a health functional food that contributes to intestinal health are divided into three categories that assist in proliferation of beneficial bacteria in the intestine, suppression of harmful bacteria, immunity regulation, and bowel movements, and there are isomaltooligosaccharide, soybean oligosaccharides and probiotics, etc. Among them, probiotics are the only raw materials for a health functional food that assist in immune regulation. However, in patients with inflammatory bowel diseases, the lining of the intestine is unstable, so the risk of developing sepsis or bacteremia is high when taking probiotics, and thus a caution is required. A study for developing a health functional food of various materials is required to resolve the risk mentioned above.

The fact that sesame oil meal has a variety of effects has already been considerably studied, but despite the possibility of using it, due to difficulties in developing an extraction process to commercialize it, sesame oil residue is still being discarded. Although some of sesame oil meals are used as feed or fertilizer, most of them are discarded, resulting in problems such as additional treatment costs, environmental pollution, etc.

As such, sesame oil meal has been expected to be fully utilized as a high value-added material, but the production of extracts has been difficult, and studies are insignificant for use as pharmaceuticals or functional food materials for the treatment of specific diseases related to this.

CN105124132A, CN104814247A, CN107501286, Elhussein et al. (BIOMASS CONVERSION AND BIOREFINERY, SPRINGER BERLIN HEIDELBERG, vol. 11, no. 4, 12 August 2019 (2019-08-12), pages 1183-1196), Akl et al. (POLYMER BULLETIN, vol. 78, no. 2, 3 March 2020 (2020-03-03), pages 1061-1085), KR20180065001A and Esmaeilzadeh (Food Science & Nutrition, vol. 2, no. 4, 1 July 2014 (2014-07-01), pages 426-435) each disclose methods for producing sesame oil meal extract comprising ultrasonic extraction of components of sesame oil cake, for example in water in water.

### [Disclosure]

### [Technical Problem]

An object of the present disclosure is to provide a method for producing a sesame oil meal extract using water as an extraction solvent.

Another object of the present disclosure is to provide a food composition for preventing or improving colitis comprising a sesame oil meal extract as an active ingredient.

Another object of the present disclosure is to provide a health functional food for preventing or improving colitis comprising a sesame oil meal extract as an active ingredient.

Another object of the present disclosure is to provide a pharmaceutical composition for preventing or treating colitis comprising a sesame oil meal extract as an active ingredient.

The technical problem to be achieved by the method for producing a sesame oil meal extract according to the technical idea of the present disclosure is not limited to the above-mentioned problem, and another problem not mentioned may be clearly understood by one skilled in the art from the following description.

### [Technical Solution]

The invention is detailed in the claims.

In order to achieve the object above, in one aspect of the invention, there is provided a method for producing a sesame oil meal extract, the method comprising the steps of: producing a sesame oil meal mixture by adding a solvent to sesame oil meal; ultrasonically extracting the sesame oil meal mixture; filtering and concentrating the ultrasonically extracted extract; and drying the concentrated concentrate, wherein the solvent is water, wherein the sesame oil meal mixture is obtained by mixing the sesame oil meal to the solvent in a weight ratio (w/v) of 1:30, wherein in the ultrasonic extraction step, an ultrasonic treatment is performed under conditions of a frequency of 10 to 50 kHz, a temperature of 20°C to 30°C, 3 to 5 hours, and an amplitude of 70 to 90%, wherein in the concentration step, after the filtration using a housing filter with a filter size between 100 µm and 50 µm, the extract is concentrated under conditions of a temperature of 50°C to 60°C, a stirring of 15 to 25 RPM, a vacuum degree of -1200 to -930 Pascal (-9 to -7 torr), and an evaporation rate of 170 to 190 L/hr, and wherein in the drying step, the concentrated concentrate is lyophilized.

According to an embodiment, the ultrasonic extraction step may be performed with a repetition of 1 to 5 times.

According to an aspect of the disclosure, the sesame oil meal mixture may be obtained by mixing the sesame oil meal to the solvent in a weight ratio (w/v) of 1:10 to 1:30.

In another aspect of the disclosure, there is provided a sesame oil meal extract produced according to the method as described above.

In another aspect of the invention, there is provided a food composition for use in preventing or improving inflammatory diseases, comprising a sesame oil meal extract produced by the method of the invention as an active ingredient, wherein the inflammatory disease is colitis accompanied with weight loss, stool abnormalities, rectal bleeding or a reduction in colon length.

According to an embodiment, the food composition for use may be a health functional food.

In another aspect of the disclosure, there is provided a health functional food for preventing or improving inflammatory diseases, comprising a sesame oil meal extract as an active ingredient.

According to an aspect of the disclosure, the health functional food may be selected from the group consisting of beverages, meat, chocolate, confectionery, pizza, ramen, other noodles, gums, candy, ice cream, alcoholic beverages, vitamin complexes, and health supplements.

According to an aspect of the disclosure, the health functional food may be one or more formulations selected from the group consisting of health functional food preparations in the form of tablets, capsules, pills, granules, liquids, powders, flakes, pastes, syrups, gels, jelly, bars, etc., beverages, gums, and candies.

In another aspect of the disclosure, there is provided a pharmaceutical composition for preventing or improving inflammatory diseases, comprising a sesame oil meal extract as an active ingredient.

According to an aspect of the disclosure, the extract may suppress weight loss, stool abnormalities, rectal bleeding and a reduction in colon length, and may have an effect of reducing the expression of inflammatory-mediated cytokines IL-6, IL-1β, and TNF-α.

According to an aspect of the disclosure, the inflammatory disease may be colitis.

According to an embodiment, the colitis may be selected from the group consisting of acute enteritis, bacterial colitis, bacterial dysentery, cholera, typhoid, traveler's diarrhea, viral colitis, pseudomembranous colitis, amoebic colitis, inflammatory bowel disease, ulcerative colitis, collagenous colitis, lymphatic colitis, ischemic colitis, convertible colitis, Crohn's disease, Behcet's syndrome, drug-induced colitis, microscopic colitis, lymphocytic colitis, radiation colitis, and indeterminate colitis.

In addition, the present invention provides a sesame oil meal extract produced by the method of the invention for use in preventing or treating inflammatory diseases wherein the inflammatory disease is colitis accompanied with weight loss, stool abnormalities, rectal bleeding or a reduction in colon length.

### [Advantageous Effects]

A sesame oil meal extract according to the present disclosure has the effect of alleviates symptoms of colitis such as weight loss, stool abnormalities, rectal bleeding, etc., inhibits a reduction in the length of the colon, and inhibiting the expression of inflammation-mediated cytokines IL-6, IL-1β, and TNF-α, and thus, a composition comprising the same as an active ingredient is a composition capable of preventing/improving or treating inflammatory diseases, particularly colitis diseases, and may be usefully used in various industrial fields such as pharmaceuticals or functional health food .

In addition, since sesame oil meal is edible as a food crop, the composition according to the present disclosure comprising an extract derived therefrom as an active ingredient has an advantage of a safety, even for long-term use. In particular, a food composition and a health functional food comprising a sesame oil meal extract have no side effects, may be taken for a long time, and may effectively suppress inflammatory bowel diseases.

However, it will be clearly understood that the effects capable of being achieved by the method for producing a sesame oil meal extract according to an embodiment of the present disclosure are not limited to those mentioned above, and other effects not mentioned are obvious to one skilled in the art from the following description.

### [Description of Drawings]

In order to more fully understand the drawings cited in the present specification, a brief description of each drawing is provided.
FIG. 1 shows a process of mass extraction of sesame oil meal by a method for producing a sesame oil meal extract according to an embodiment of the present disclosure.
FIG. 2 shows the measurement of weight of a DSS colitis mouse model according to an embodiment of the present disclosure.
FIG. 3 shows the measurement of an amount of drinking water of the DSS colitis mouse model according to an embodiment of the present disclosure.
FIG. 4 shows the measurement of a degree of stool abnormality of the DSS colitis mouse model according to an embodiment of the present disclosure.
FIG. 5 shows the measurement of a degree of rectal bleeding of the DSS colitis mouse model according to an embodiment of the present disclosure.
FIG. 6 shows the measurement of a length of large intestine of the DSS colitis mouse model according to an embodiment of the present disclosure.
FIG. 7 shows the result of H&E stain of large intestine tissue of the DSS colitis mouse model according to an embodiment of the present disclosure.
FIG. 8 shows the result of measuring IL-6 of the DSS colitis mouse model according to an embodiment of the present disclosure.
FIG. 9 shows the result of measuring IL-1β of the DSS colitis mouse model according to an embodiment of the present disclosure.
FIG. 10 shows the result of measuring IFN-γ of the DSS colitis mouse model according to an embodiment of the present disclosure.
FIG. 11 shows the result of measuring TNF-α of the DSS colitis mouse model according to an embodiment of the present disclosure.

### [Best Mode]

The present disclosure provides a method for producing a sesame oil meal extract, the method comprising the steps of: producing a sesame oil meal mixture by adding a solvent to sesame oil meal; ultrasonically extracting the sesame oil meal mixture; filtering and concentrating the ultrasonically extracted extract; and drying the concentrated concentrate, wherein the solvent is water, wherein the sesame oil meal mixture is obtained by mixing the sesame oil meal to the solvent in a weight ratio (w/v) of 1:30, wherein in the ultrasonic extraction step, an ultrasonic treatment is performed under conditions of a frequency of 10 to 50 kHz, a temperature of 20°C to 30°C, 3 to 5 hours, and an amplitude of 70 to 90%, wherein in the concentration step, after the filtration using a housing filter with a filter size between 100 µm and 50 µm, the extract is concentrated under conditions of a temperature of 50°C to 60°C, a stirring of 15 to 25 RPM, a vacuum degree of -1200 to -930 Pascal (-9 to -7 torr), and an evaporation rate of 170 to 190 L/hr, and wherein in the drying step, the concentrated concentrate is lyophilized.

In the present disclosure, "sesame oil meal" is a by-product obtained from sesame, excluding sesame oil after a pressing process. As the sesame, it is possible to use, without limitation, sesame that has been heat-treated at low or high temperature, or raw sesame that has not undergone a heating process.

According to the invention, water is used as a solvent capable of efficiently extracting sesame oil meal. When water is used as a solvent, there is an advantage in that since the use of an expensive ethanol solvent is not required, and an economic cost burden for a heating process for ethanol vaporization, for an additional process for neutralization, and for wastewater treatment is reduced.

In the present disclosure, the term "extract" also includes a fraction obtained by further fractionating the extract. That is, the fraction re-fractionated with solvents, or the fraction obtained through various chromatographies or an ultrafiltration membrane having a constant molecular weight cut-off value as well as extracts of water, lower alcohols having 1 to 4 carbon atoms, nonpolar solvents, or mixed solvents thereof are included.

According to the invention, in the ultrasonic extraction step, an ultrasonic treatment is performed under conditions of a frequency of 10 to 50 kHz, a temperature of 20°C to 30°C, 3 to 5 hours, and an amplitude of 70 to 90%.

Conventionally, processes such as hot water extraction, ultrasonic extraction, etc., are used as a method for obtaining physiologically active substances from natural products. However, the optimum extraction conditions depend on the characteristics of the target substances, and thus, it is necessary to establish the optimum extraction conditions according to the target substances.

On the other hand, the conventional ultrasonic extraction process is a very low economical process since the use of alcohol and acid incurs an environmental cost burden due to neutralization and wastewater treatment, and a long extraction time is required.

The method for producing the sesame oil meal extract according to the present disclosure does not use any organic solvent and uses only water, so there is no need for an additional process for neutralization, and the economic cost burden for wastewater treatment may be reduced. Specifically, it is to be performed under the optimum extraction conditions for the production of a large amount of sesame oil meal extract according to the present disclosure, and for maintaining stability and maintaining the physiological activity function of the extract at a frequency of 10 to 50 kHz, a temperature of 20°C to 30°C, 3 to 5 hours and an amplitude of 70 to 90%, and preferably, a frequency of 20 kHz, a temperature of 25°C, 4 hours, and an amplitude of 80%.

According to an embodiment, the ultrasonic extraction step may be performed by repeating 1 to 5 times, and preferably, ultrasonic extraction may be performed by repeating 3 to 5 times.

According to the invention, the sesame oil meal mixture is obtained by mixing the sesame oil meal to the solvent in a weight ratio (w/v) of 1:30. Specifically, it is mixed by adding water including purified water of about 30 times (w/v) the weight of the sesame oil meal to the dried sesame oil meal.

According to an embodiment, the ultrasonically-extracted extract is lyophilized after the filtration using a housing filter (with a filter size between 100 µm and 50 µm).

According to an embodiment, the concentration conditions may be performed under conditions of a temperature of 50°C to 60° C, a stirring of 15 to 25 RPM, a vacuum degree of -9 to -7 torr, and an evaporation rate of 170 to 190 L/hr, and preferably, it may be carried out under the conditions of a temperature of 55°C to 60°C, a stirring of 20 RPM, a vacuum degree of -8 torr, and an evaporation rate of 180 L/hr.

According to an embodiment, in the concentration method, the filtrate is transferred to a stirring type concentrator using a diaphragm pump, and concentration is started under the concentration condition after the transfer is completed. Thereafter, when the concentration is completed, it is pressurized and transferred to a liquid mobile tank.

According to an embodiment, the drying may be performed by a method such as freeze drying, spray drying, low temperature cold air drying, etc. Preferably, it may be carried out at a temperature of -50°C to -40°C, freezing conditions of 5 to 7 hours; a temperature of 25°C to 35°C, and drying conditions of 48 to 52 hours, and more preferably, at a temperature of -45°C, freezing conditions of 6 hours; a temperature of 30°C, and a drying condition of 51 hours.

According to an embodiment, in the drying method, after setting a tray on a shelf, the concentrate is put in a beaker, and then is frozen and dried under the above conditions.

In addition, disclosed herein but not part of the invention is a sesame oil meal extract produced according to the method. The sesame oil meal extract has an effect of suppressing or improving the symptoms of colitis.

In addition, the present disclosure provides a food composition comprising a sesame oil meal extract produced by the method of the invention for use in preventing or improving inflammatory diseases, wherein the inflammatory disease is colitis accompanied with weight loss, stool abnormalities, rectal bleeding or a reduction in colon length .

According to an embodiment, the food may be a health functional food.

In the present disclosure, the term "prevention" refers to any action of inhibiting or delaying the onset of a specific disease (for example, colitis) by administration of a composition comprising the sesame oil meal extract according to the present disclosure.

As used herein, the term "improvement" refers to any action which at least reduces a parameter related to the condition being treated, for example, the severity of symptoms.

In the present disclosure but not part of the invention, examples of "inflammatory disease" may include systemic lupus erythematosus, scleroderma, ulcerative colitis, Crohn's disease, atopic dermatitis, psoriasis, anaphylaxis, dermatitis, diabetic retinopathy, retinitis, macular degeneration, uveitis, conjunctivitis, stroke, coronary artery disease, arteriosclerosis, atherosclerosis, myocardial infarction, unstable angina, vasculitis, vascular stenosis, Kawasaki disease, giant cell arteritis, arthritis, rheumatoid arthritis, ankylosing spondylitis, osteoarthritis, osteoporosis, allergy, diabetes, diabetic renal disease, nephritis, nephritis, Sjogren's syndrome, autoimmune pancreatitis, periodontal disease, asthma, graft versus host disease, chronic pelvic inflammatory disease, endometritis, rhinitis, tumor metastasis, transplant rejection, and chronic prostatitis, etc.

In the present disclosure but not part of the invention, the term "colitis" refers to a state in which inflammation has occurred in the large intestine, for example, may include, but is not limited to, acute enteritis, bacterial colitis, bacterial dysentery, cholera, typhoid, traveler's diarrhea, viral colitis, pseudomembranous colitis, amoebic colitis, Inflammatory bowel disease, ulcerative colitis, collagenous colitis, lymphoid colitis, ischemic colitis, convertible colitis, Crohn's disease, Behcet's syndrome, drug-induced colitis, microscopic colitis, lymphocytic colitis, radiation colitis or indeterminate colitis.

In the present disclosure, the term "food composition" refers to food which provides a better health condition by acting advantageously on one or more functions of the organism, regardless of the nutrients provided to the subject ingesting the same. Consequently, the food composition may be used for the prevention, improvement, or treatment of diseases or disease-causing factors.

The food composition according to the present disclosure is a food composition comprising a sesame oil meal extract as an active ingredient. According to an embodiment, the sesame oil meal extract may be included in an amount of 0.001 to 99.9% by weight based on the total weight of the composition.

In addition to comprising the sesame oil meal extract as an active ingredient, the food composition according to the present disclosure may comprise various flavoring agents or natural carbohydrates as an additional ingredient, like a conventional food composition.

Examples of the aforementioned natural carbohydrates are monosaccharides, for example, glucose, fructose, etc.; disaccharides, for example, as maltose, sucrose, etc.; and polysaccharides, for example, common sugars such as dextrin and cyclodextrin, and sugar alcohols such as xylitol, sorbitol, and erythritol. The flavoring agent as described above may be advantageously used as a natural flavoring agent (taumatin), a stevia extract (for example, rebaudioside A, glycyrrhizin, etc.), and a synthetic flavoring agent (saccharin, aspartame, etc.).

In addition, the food composition according to the present disclosure may be preferably formulated as a food composition by comprising at least one sitologically acceptable or pharmaceutically acceptable carrier in addition to the active ingredients as described above.

The formulation form of the food composition may be a tablet, capsule, powder, granule, liquid, pill, liquid, syrup, juice, suspension, emulsion, or drop. For example, for formulation in the form of tablets or capsules, the active ingredient may be combined with an oral, non-toxic pharmaceutically acceptable inert carrier such as ethanol, glycerol, water, etc. In addition, if desired or necessary, suitable binders, lubricants, disintegrants, and coloring agents may also be included in the mixture. Suitable binders are, but are not limited to, natural sugars such as starch, gelatin, glucose, or beta-lactose, corn sweeteners, natural and synthetic gums such as acacia, tragacanth, or sodium oleate, sodium stearate, magnesium stearate, sodium benzoate, sodium acetate, sodium chloride, etc. Disintegrants include, but are not limited to, starch, methyl cellulose, agar, bentonite, xanthan gum, etc. As pharmaceutical carriers acceptable for compositions formulated as liquid solutions, which are suitable for sterilization and biocompatibility, saline, sterile water, Ringer's solution, buffered saline, albumin injection solution, dextrose solution, maltodextrin solution, glycerol, ethanol, and one or more of these components may be mixed and used, and other conventional additives such as antioxidants, buffers, and bacteriostatic agents may be added, if necessary. In addition, diluents, dispersants, surfactants, binders, and lubricants may be additionally added to prepare injectable formulations such as aqueous solutions, suspensions, emulsions, etc., pills, capsules, granules, or tablets.

The food composition the food composition according to the present disclosure prepared in the above manner may be used as a functional food or added to various foods.

Foods to which the composition according to the present disclosure may be added include, for example, beverages, meat, chocolate, foods, confectionery, pizza, ramen, other noodles, gums, candy, ice cream, alcoholic beverages, vitamin complexes, and health supplements, etc.

In addition, the food composition may include various nutrients, vitamins, minerals (electrolytes), flavoring agents such as synthetic flavors and natural flavoring agents, coloring agents and thickeners (cheese, chocolate, etc.), pectic acid and a salt thereof, alginic acid and a salt thereof, organic acids, protective colloidal thickeners, pH adjusters, stabilizers, preservatives, glycerin, alcohols, carbonizing agents used in carbonated beverages, etc. In addition, the food composition according to the present disclosure may contain flesh for the production of natural fruit juice, and fruit juice beverage and vegetable beverage.

The sesame oil meal extract, which is an active ingredient of the present disclosure, is a natural substance and has almost no side effects such as those caused by chemicals, so it may be safely used for long-term administration for the purpose of imparting antioxidant functionality.

In addition as disclosed herein but not part of the invention is provided a food composition comprising a sesame oil meal extract obtained according to the method of the invention as an active ingredient for use in preventing or improving an inflammatory disease wherein the inflammatory disease is colitis accompanied with weight loss, stool abnormalities, rectal bleeding or a reduction in colon length.

The food composition according to the present disclosure has no side effects, may be taken for a long time, and has an inhibitory effect on inflammatory diseases. In addition, the present disclosure provides a health functional food for preventing or improving inflammatory diseases, comprising a sesame oil meal extract as an active ingredient.

The health functional food according to the present disclosure may be produced and processed in the form of tablets, capsules, powders, granules, liquids, pills, etc. for the purpose of preventing or improving inflammation-related diseases.

In the present disclosure, the term "health functional food" refers to food preparation and processed using raw materials or ingredients having useful functions for the human body in accordance with the Health Functional Food Act, and means ingestion for the purpose of controlling nutrients for the structure and function of the human body or obtaining a useful effect for health use such as physiological action.

The health functional food according to the present disclosure may comprise ordinary food additives, and whether it is suitable as food additives is judged by determining standards and criteria for relevant items according to the general rules and general test methods, etc. for food additives code approved by the Food and Drug Administration, unless otherwise specified.

Examples of the items listed in the food additive code include chemical compounds such as ketones, glycine, calcium citrate, nicotinic acid, and cinnamic acid; natural additives such as persimmon color, licorice extract, crystalline cellulose, kaoliang color, and guar gum; mixed preparations, such as a sodium L-glutamate preparation, an alkali additive for noodles, a preservative preparation, and a tar color preparation, etc.

For example, in a health functional food in the form of tablets, a mixture of a sesame oil meal extract as an active ingredient of the present disclosure, mixed with excipients, binders, disintegrants, and other additives, is granulated by a conventional method, and then is molded by a compression with an addition of a lubricant, etc., or the mixture may be directly molded with a compression. In addition, the health functional food in the form of tablets may contain a mating agent, etc., if necessary.

Among the health functional foods in the form of capsules, hard capsules may be prepared by filling a mixture of a sesame oil meal extract as an active ingredient of the present disclosure with additives such as excipients, in a conventional hard capsule, and soft capsules may be prepared by filling a mixture of the extract with additives such as excipients in a capsule base such as gelatin. The soft capsules may contain a plasticizer such as glycerin or sorbitol, a colorant, a preservative, etc., if necessary.

The health functional food in the form of pills may be prepared by molding a mixture of a sesame oil meal extract as an active ingredient of the present disclosure, with excipients, binders, disintegrants, etc., with a known method, and may be coated with white sugar or the other coating agent, if necessary, or the surface may be coated with a material such as starch or talc.

The health functional food in the form of granules may be prepared in granular form by mixing sesame oil meal extract as an active ingredient of the present disclosure, with excipients, binders, disintegrants, etc., according to a conventionally known method and may contain flavoring agents, mating agents and the like, if necessary.

The sesame oil meal extract according to the present disclosure has been experimentally proven to have an excellent anti-inflammatory, particularly anti-colitis effect, and is effective in preventing or improving inflammation-related diseases.

According to an embodiment, the health functional food may be beverages, meat, chocolate, confectionery, pizza, ramen, other noodles, gums, candy, ice cream, alcoholic beverages, vitamin complexes, and health supplements.

According to an embodiment, the health functional food may be one or more formulations selected from the group consisting of health functional food preparations in the form of tablets, capsules, pills, granules, liquids, powders, flakes, pastes, syrups, gels, jelly, bars, etc., beverages, gums, and candies.

In addition, the present disclosure provides a health functional food comprising a sesame oil meal extract produced according to the method of the invention as an active ingredient for use in preventing or improving inflammatory diseases, wherein the inflammatory disease is colitis accompanied with weight loss, stool abnormalities, rectal bleeding or a reduction in colon length.

The health functional food according to the present disclosure has no side effects, may be taken for a long time, and has an inhibitory effect on inflammatory diseases.

In addition, the present disclosure provides a pharmaceutical composition for use in preventing or treating colitis accompanied with weight loss, stool abnormalities, rectal bleeding or a reduction in colon length, comprising a sesame oil meal extract produced according ot the method of the invention as an active ingredient.

According to the disclosure and not part of the invention, a pharmaceutical composition for preventing or treating colitis, comprising a sesame oil meal extract as an active ingredient may be prepared by including a sesame oil meal extract and a pharmaceutically acceptable salt, and may be used for the treatment and prevention of colitis, including weight loss, stool abnormalities, and an inhibition of rectal bleeding and a reduction in the length of the large intestine.

In the present disclosure, the term "pharmaceutically acceptable salt" means a salt prepared by a conventional method, and may be used, without limitation, by a method known to one skilled in the art. The pharmaceutically acceptable salts include, but are not limited to, pharmacologically or physiologically acceptable salts derived from the following inorganic and organic acids and bases. Examples of suitable acids may include hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, naphthalene-2-sulfonic acid, benzenesulfonic acid, etc. Salts derived from suitable bases may include alkali metals such as sodium, alkali earth metals such as magnesium, ammonium, etc.

In the present disclosure, the term "treatment" refers to any action of ameliorating or beneficially changing the symptom of a specific disease (for example, colitis) by administration of a composition comprising the sesame oil meal extract according to the present disclosure.

In the present disclosure, the term "administration" means introducing the composition of the present disclosure to a patient by any suitable method, and the route of administration of the composition of the present disclosure may be various oral or parenteral routes as long as it can reach the target tissue, and may be administered in a conventional mode by specifically, oral, rectal, topical, intravenous, intraperitoneal, intramuscular, intraarterial, transdermal, intranasal, inhalation, intraocular or intradermal routes. The treatment method of the present disclosure includes administering the composition of the present disclosure in a pharmaceutically effective amount. It is obvious to those skilled in the art that an appropriate amount to be total daily used may be determined by the treating physician within the range of correct medical judgment. The specific therapeutically effective amount for a specific patient is preferable to be differently applied, depending on the type and extent of the reaction to be achieved, the specific composition, including whether or not other agents are used in some cases, the patient's age, weight, general health status, sex and diet, administration time, various factors including the route of administration and the secretion rate of the composition, the treatment period, drugs used with or concurrently with the specific composition, and similar factors well known in the medical field. Therefore, it is preferable to determine an effective amount of a composition suitable for the purposes of the present disclosure, consideration the foregoing.

According to an embodiment, the sesame oil meal extract may be included in an amount of 0.001 to 99.9% by weight based on the total weight of the composition.

In addition, the composition according to the present disclosure may be applied to any animal in need of treatment and prevention of colitis diseases, and the animal includes not only humans and primates, but also livestock such as cattle, pigs, sheep, horses, dogs and cats, etc.

The pharmaceutical composition according to the present disclosure may be administered orally or parenterally during clinical administration, and may be used in the form of a general pharmaceutical formulation.

The pharmaceutical composition may be characterized in that it is formulated in the form of powders, granules, tablets, hard capsules, soft capsules, or injection.

More specifically, it may be formulated in the form, but is not limited to, oral dosage forms such as powders, granules, tablets, capsules, suspensions, emulsions, syrups, aerosols, etc., external preparations, suppositories, sterile injection solutions, pre-filled injection solutions, or lyophilized form, according to each conventional method.

In the case of formulation, it may be prepared using diluents or excipients such as commonly used fillers, extenders, binders, wetting agents, disintegrants, and surfactants.

Solid preparations for oral administration include tablets, pills, powders, granules, capsules, etc, and such solid preparations may be prepared by mixing at least one excipient, such as starch, calcium carbonate, sucrose, lactose, gelatin, etc. in the active ingredient. Also, in addition to simple excipients, lubricants such as magnesium stearate and talc may be used.

Liquid formulations for oral administration include suspensions, liquid solutions, emulsions, syrups, etc., and in addition to water, and liquid paraffin as simple diluents commonly used, various excipients such as wetting agents, sweeteners, fragrances, preservatives, etc. may be included. Formulations for parenteral administration include sterile aqueous solutions, non-aqueous solvents, suspensions, emulsions, lyophilized formulations, suppositories, and the like.

A preferred dosage of the pharmaceutical composition according t the present disclosure may be appropriately selected according to the condition and weight of the patient, the degree of symptoms, the form of the drug, the route and duration of administration.

In addition, the present disclosure provides sesame oil meal extract produced by the method of the invention for use in the prevention or treatment of inflammatory diseases, wherein the inflammatory disease is colitis accompanied with weight loss, stool abnormalities, rectal bleeding or a reduction in colon length.

The sesame oil meal extract according to the present disclosure suppresses body weight loss, stool abnormalities, rectal bleeding, etc., and suppresses a reduction in the length of the large intestine. In addition, the extract may have an effect of reducing the expression of inflammation-mediated cytokines, IL-6, IL-1β, and TNF-α.

The present disclosure measured body weight, an amount of drinking water, stool abnormality, a degree of rectal bleeding, a length of the large intestine, tissue staining, and cytokines, in order to confirm the anti-colitis efficacy of a sesame oil meal ultrasound extract in a colitis mouse model by using dextran sodium sulfate (DSS). The positive control used chlorogenic acid, which is known to be effective in anti-colitis. According to an embodiment of the present disclosure, the effect of the sesame oil meal ultrasound extract was able to be confirmed as suppressing or improving colitis symptoms than a DSS-alone treatment group.

The main symptoms of colitis in mice such as weight loss, contraction of the large intestine, mucosal ulcers, etc. occur, resulting in symptoms similar to those of humans. In an example of the present disclosure, DSS was used to induce inflammatory bowel disease in mice. DSS induces inflammatory reactions in the mucous membrane by changing an intestinal flora, and in the early stages of colitis, it causes bloody stool, weight loss, and a reduction in the length of the large intestine. As colitis progresses chronically, various immune responses occur in the mucous membrane, causing intestinal inflammation. In an example of the present disclosure, sesamin and sesamolin among the components of the sesame oil meal extract were set as indicators to test whether there is anti-colitis effect in a DSS-induced colitis mice model together with the sesame oil meal extract.

According to an embodiment of the present disclosure, the DSS-alone treatment group showed body weight loss, stool abnormality, rectal bleeding, a reduction in the large intestine, tissue necrosis, and an increase in inflammatory cytokines, compared to the CON (normal) group. By contrary, in the group treated with the sesame oil meal extract, body weight loss was suppressed, and stool abnormalities, rectal bleeding, and reduction in the large intestine were suppressed, compared to the group treated with DSS alone.

In general, DSS is known to have a direct toxic effect on epithelial cells and induce activation of macrophages and T cells, thereby increasing both Th1 and Th2 cytokines and other inflammatory mediators to induce colitis. IL-6, IL-1β, and TNF-α, which are inflammation-mediated cytokines involving Th1 and Th2, were found to decrease in the group treated with the sesame oil meal extract, and in particular, TNF-α as cytokine was significantly reduced. Through this, it is thought that the sesame oil meal extract has the potential to exhibit anti-inflammatory effects through a Th1 pathway. As the inflammatory response of the large intestine decreases, it may be said that the reduction in the length of the large intestine is suppressed and tissue necrosis is reduced. Tissue necrosis was reduced, and stool abnormalities and anal bleeding were also reduced, and the anti-inflammatory effect of the large intestine was effective in suppressing body weight loss.

Therefore, it can be said that the sesame oil meal extract according to the present disclosure has the potential to improve inflammatory bowel disease and assist in bowel health via anti-inflammatory effects through the Th1 pathway.

In addition, the sesame oil meal extract according to the present disclosure has high utility value as a functional raw material that assists in intestinal health. Specifically, the sesame oil meal extract according to the present disclosure may effectively suppress inflammatory bowel diseases by controlling the immune response in the intestine compared to the conventional individually recognized raw materials such as isomaltoligosaccharide and soybean oligosaccharide, and has the advantage of long-term use without risk of various side effects of probiotics (lactic acid bacteria) reported in patients with weak immunity.

Hereinafter, examples and experimental examples will be described in detail in order to describe the present disclosure in detail. However, the examples and experimental examples according to the present disclosure may be modified in various different forms, and the examples and experimental examples described below are provided to explain the present disclosure completely to those with average knowledge in the art.

### Examples

### 1. Material and Method

### 1.1 Production of sesame oil meal ultrasonic extracts

The sesame used in the present disclosure was produced through contract cultivation with farmers in Gochang, Jeonbuk by receiving seeds from the National Institute of Food Science and Technology. The sesame was washed and oil was separated using a screw-type press at a temperature of 40 to 70°C, and then sesame meal was used as a sample.

1 ton of water was added to the obtained 33 kg of sesame oil meal and mixed using a stirrer. The ultrasonic oscillator and circulation pump were operated, and the extraction process was started under the conditions of an alcohol concentration of 0%, a frequency of 20 kHz, a temperature of 25°C, and four (4) hours and an amplitude of 80%, and a part of the sample was then sampled for analysis of the extract. At this time, a part of the sample was discharged through a discharge pump. Thereafter, the ultrasonically extracted extract was filtered through a filter cloth, and a part was filtered using a housing filter (100 µm, 50 µm).

The filtrate was transferred to a stirring type concentrator using a diaphragm pump (filtered by using bag filters (20 mesh, 40 mesh) due to sludge, and the sludge deposited at the bottom of the barrel was not transferred), and after the transfer was completed concentration was started under the conditions of a temperature of 55°C to 60°C, a stirring of 20 RPM, a vacuum degree of -8 torr, and an evaporation rate of 180 L/hr. After checking the liquid amount, concentration was completed when the liquid amount reached around 200L and reached the bolt under the rubber parking at the bottom of the stirrer. It was then pressurized and transferred to a liquid mobile tank. The equipment was run by cleaning-in-place (CIP) (caustic soda).

Thereafter, after setting the tray on the shelf, the concentrate was added while adding 2 to 2.5L of the concentrate to the beaker at a time, and then the temperature sensor was checked and sampled. The freezing conditions were set to a temperature of -45°C and 6 hours, and the drying conditions were set to a temperature of 30°C and 51 hours, and the equipment was then operated. After the lyophilization was completed, the raw material was recovered in a vinyl for subdivision (large). The equipment was run by CIP (tray, main body, washing with high pressure water).

### 1.2 Production of comparative examples

In order to search for an optimal sesame oil meal extract by extraction method, hot water extract and room temperature extract of sesame oil meal were produced as comparative examples.

(Comparative Example 1: hot water extraction) Sesame oil meal to water was mixed in a ratio of 1:30, extracted with hot water at 100°C for 4 hours, and then left to cool at room temperature for 30 minutes.

(Comparative Example 2: room temperature extraction) Sesame oil meal and water were mixed in a ratio of 1:30 and extracted for 4 hours at room temperature (25°C).

The hot water extract and room temperature extract were filtered using a vacuum filtration system and a filter paper (Hyundai Micro, KOREA), and then concentrated to 1/4 (v/v) using a vacuum rotary concentrator. Thereafter, the concentrate was dried for 4 days using a freeze dryer (Ilshin, KOREA) to form a powder, and Comparative Examples 1 and 2 were produced.

### 2. Efficacy evaluation of sesame oil meal extract

### 2.1 Experimental animal and administration method

Eight (8)-week-old C57BL/6 male mice were acclimatized for a week and then used in the experiment. During the experiment, feed and drinking water were freely ingested, and the temperature of the breeding room was maintained at 22±2°C, the humidity was 55±15%, and the light/dark cycle was maintained for 12 hours. Sesamolin (Sigma aldrich, USA) and sesamolin (Sigma aldrich, USA) were used as standard material of sesamin oil meal. The experimental group was ten (10) mice of the normal group to which distilled water was administered, ten (10) mice of the DSS-alone treatment group administered with 2.5% DSS (dextran sodium sulfate, MPBIO, CANADA) in drinking water, and each ten (10) mice of low, medium, and high Treatment groups administered with 10, 100, 1000 mg/kg sesame oil meal ultrasonic extract with 2.5% DSS, respectively, each ten (10) mice of the sesamin and sesamolin treatment groups administered with 120 mg/kg of Sesamin, and 120 mg/kg sesamolin with 2.5% DSS, ten (10) positive control administered with 200 mg/kg chlorogenic acid with 2.5% DSS and were divided into 8 groups and administered once a day. The test period was set to 8 days in which 20% of the body weight of the mice was lost based on the results of the preliminary experiment (Table 1).

**[Table 1]**

| Set of the experimental group | | | |
|---|---|---|---|
| Num ber | Experiment al group | Administration material | Frequency and route for an administration |
| 1 | CON (normal group) | Distilled water | DSS: freely approaching the free drinking water feeding sample/PC: for eight (8) days oral administration one time for 1 day |
| 2 | 2.5% DSS | 2.5% DSS | |
| 3 | 10 | 2.5% DSS+ sesame oil meal ultrasonic extract (10 mg/kg) | |
| 4 | 100 | 2.5% DSS+ sesame oil meal ultrasonic extract (100 mg/kg) | |
| 5 | 1000 | 2.5% DSS+ sesame oil meal ultrasonic extract (100 mg/kg) | |
| 6 | Sesamin | 2.5% DSS+Sesamin (120 mg/kg) | |
| 7 | Sesamolin | 2.5% DSS+Sesamolin (120 mg/kg) | |
| 8 | PC (Positive control) | 2.5% DSS+Chlorogenic acid (200 mg/kg) | |

### 2.2 Production of a sample

The extract powder was stored at room temperature, and immediately before oral administration, was dissolved in primary distilled water containing 5% dimethyl sulfoxide (Sigma aldrich, USA).

### 2.3 Measurement of symptom of colitis

In each experimental group, mouse body weight, an amount of drinking water, a degree of stool abnormality, and a degree of rectal bleeding according to the induction of colitis were measured. Mouse body weight and drinking water were measured at the same time every day. The degree of stool abnormality was determined by placing in an individual cage on the 8th day of DSS administration and observing stool condition with an induction of defecation. The score was calculated and digitalized by referring to the measurement method of Kim et al., 2013, Gastroenterology paper, based on the degree of watery stool and the presence or absence of blood stool. The degree of rectal bleeding was quantified by performing a fecal occult blood test using a Hema-screen kit to measure the degree of bleeding in the stool and anus immediately after defecation on the 8th day of DSS administration.

### 2.4 Measurement of the length of the large intestine

The length of the large intestine according to the induction of colitis in each experimental group was measured. On the 8th day of DSS administration, the experimental animals were sacrificed, and the organs from the cecum to the anus were removed through autopsy, photographs were taken, and the length of the large intestine was measured.

### 2.5 Histological examination of the large intestine

Histological examination was performed to observe the degree of the large intestine lesions such as thickening of mucosal tissues, redness, an increase in inflammatory cells, and ulcers due to tissue necrosis according to the induction of colitis in each experimental group. After the animals were sacrificed on the 8th day of DSS administration, organs from the cecum to the anus were removed through autopsy, washed with phosphate-buffer saline (PBS, Gibco, USA) and fixed in 4% formaldehyde for 2 days. Thereafter, a tissue slide was prepared and the tissue was observed through Hematoxylin & Eosin (H&E) staining.

### 2.6 Measurement of pro-inflammatory cytokine (IL-6, IL-1β, IFN-γ, TNF-α)

ELISA was performed to observe the degree of pro-inflammatory cytokines according to the induction of colitis in each experimental group. On the 8th day of DSS administration, the experimental animals were sacrificed, the organs from the cecum to the anus were removed through autopsy, washed with PBS, and lysis buffer was then added to dissolve the tissue, and the supernatant was collected through centrifugation.

Concentration of Interleukin (IL)-6 in the tissue was measured by ELISA. The supernatant obtained by dissolving the tissue was used as a sample, 50 uL of assay diluent RD1-14 was added to each well, and 50 uL of each of the standard, sample, and control were added and reacted at room temperature for 2 hours. Thereafter, 100 uL of mouse IL-6 conjugate was added, 2 hours at room temperature was maintained, 100 uL of a substrate solution was added to react at room temperature for 30 minutes under light-shielding condition, and then 50 uL of a stop solution was added, and absorbance at 450 nm using a spectrophotometer was measured. Between each process, washing of each well was performed 3 times using washing buffer.

Concentration of Interleukin (IL)-1β in the tissue was measured by ELISA. The supernatant obtained by dissolving the tissue was used as a sample, 50 uL of assay diluent RD1N was added to each well, and 50 uL of each of the standard, sample, and control were added and reacted at room temperature for 2 hours. Thereafter, 100 uL of mouse IL-1β conjugate was added, 2 hours at room temperature was maintained, 100 uL of a substrate solution was added to react at room temperature for 30 minutes under light-shielding condition, and then 50 uL of a stop solution was added, and absorbance at 450 nm using a spectrophotometer was measured. Between each process, washing of each well was performed 3 times using washing buffer.

Concentration of Interleukin (IFN)-γ in the tissue was measured by ELISA. The supernatant obtained by dissolving the tissue was used as a sample, 50 uL of assay diluent RD1-21 was added to each well, and 50 uL of each of the standard, sample, and control were added and reacted at room temperature for 2 hours. Thereafter, 100 uL of mouse IFN-γ conjugate was added, 2 hours at room temperature was maintained, 100 uL a substrate solution was added to react at room temperature for 30 minutes under light-shielding condition, and 50 uL of a stop solution was then added, and absorbance at 450 nm using a spectrophotometer was measured. Between each process, washing of each well was performed 3 times using washing buffer.

Concentration of tumor necrosis factor (TNF)-α in the tissue was measured by ELISA. The supernatant obtained by dissolving the tissue was used as a sample, 50 uL of assay diluent RD1-63 was added to each well, and 50 uL of each of the standard, sample, and control were added and reacted at room temperature for 2 hours. Thereafter, 100 uL of mouse TNF-α conjugate was added, 2 hours at room temperature was maintained, 100 uL of a substrate solution was added to react at room temperature for 30 minutes under light-shielding condition, and 50 uL of a stop solution was then added, and absorbance at 450 nm using a spectrophotometer was measured. Between each process, washing of each well was performed 3 times using washing buffer.

### 2.7 Statistical analysis

All results of the experiment were expressed as mean ± standard error. In order to verify the significance of each group, a one-way analysis of variance (ANOVA) test was performed, and statistical significance was represented by p* <0.05, p** <0.005, p*** <0.001 vs CON, p# <0.05, p## <0.005, p### <0.001 vs DSS.

### 3. Result analysis

### 3.1 Result of producing the sesame oil meal extract

Using sesame oil meal provided by Quen's Bucket, a hot water extract (Comparative Example 1) and a room temperature extract (Comparative Example 2) were prepared, and the final yield after lyophilization was completed as 8.1% for a hot water extraction and 7.8% for a room temperature extraction.

### 4. Efficacy evaluation of sesame oil meal extract

### 4.1 Measurement of symptom of colitis

### 4.1.1 Measurement of mouse body weight

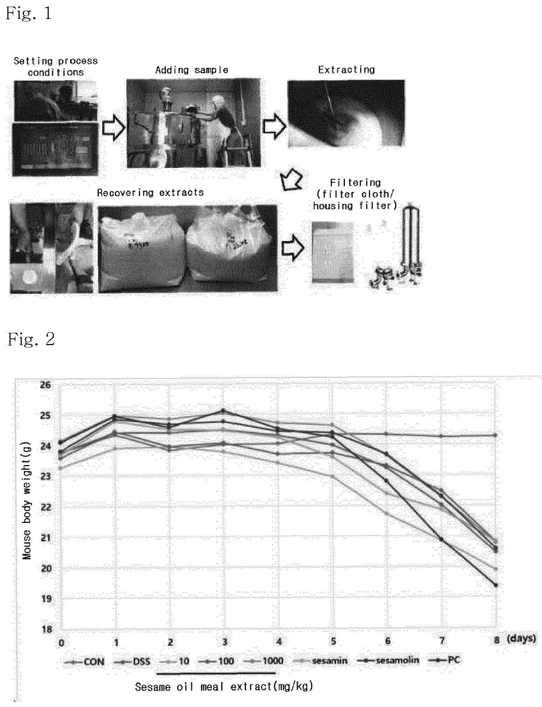

Body weight was measured every day to determine whether the sesame oil meal extract has anti-colitis effect. Referring to FIG. 2, as a result of body weight measurement, when compared with the DSS-alone treatment group, the treatment group of medium concentration (100 mg/kg) of the sesame oil meal extract showed a tendency to inhibit mouse body weight loss from the 5th day, but the body weight on the 8th day was no significant change.

### 4.1.2 Measurement of amount of drinking water of mouse

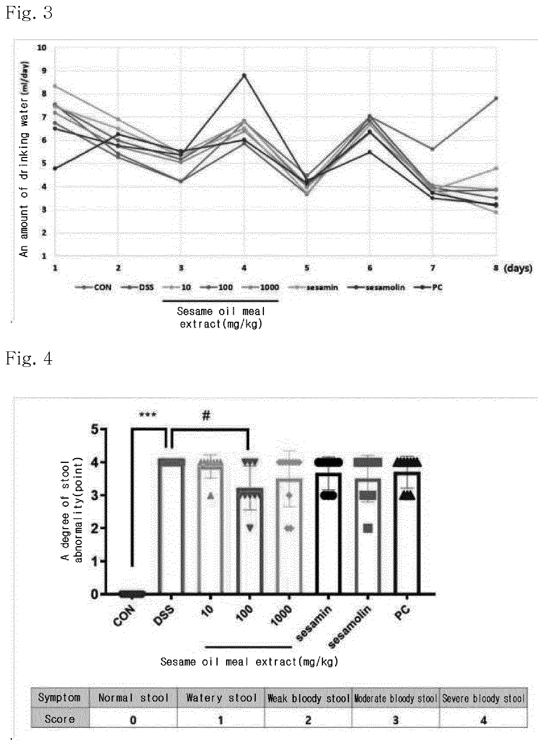

Referring to FIG. 3, as a result of measuring the amount of drinking water, when compared with the DSS-alone treatment group, the treatment group of the sesame oil meal extract did not show a significant difference. Based on the results of this experiment, it can be seen that the induction of colitis caused by DSS through the same amount of drinking water showed no difference between groups.

### 4.1.3 Measurement of degree of stool abnormality

The degree of stool abnormality in mice on the 8th day of the experiment was measured to determine whether the sesame oil meal extract has anti-colitis effect. It is known that when colitis is caused by DSS, watery stools are excreted, and when symptoms become severe, bloody stools are excreted. Referring to FIG. 4, as a result of the measurement, all mice in the DSS-alone treatment group showed bloody stool, and the mice with bloody stool symptoms were significantly reduced in the treatment group of the medium concentration (100 mg/kg) of the sesame oil seed extract.

### 4.1.4 Measurement of degree of rectal bleeding

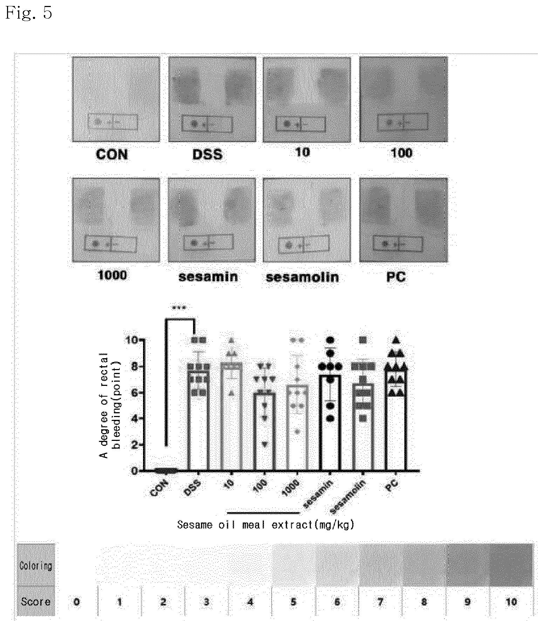

To find out if sesame oil meal extract has anti-colitis effect, rectal bleeding that occurs during inflammatory reactions in the large intestine was measured. Referring to FIG. 5, as a result of the measurement, when compared with the CON (normal) group, all groups administered with DSS had increased rectal bleeding symptoms. Compared with the DSS-alone treatment group, the degree of rectal bleeding decreased in the treatment group of the medium concentration (100 mg/kg) of the sesame oil seed extract.

### 4.2 Measurement of the length of the large intestine

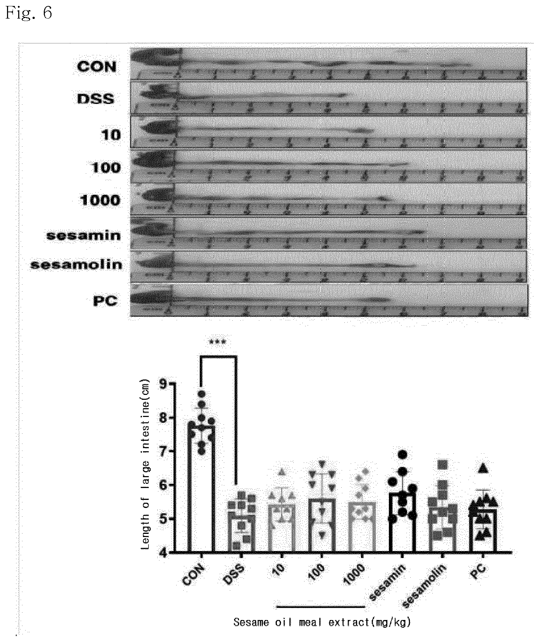

In order to determine whether sesame oil meal extract has anti-colitis effect, mice were autopsied on the 8th of the experiment, and the length from the cecum to the anus was measured. It is known that excessive inflammatory reactions in the large intestine shorten the length. Referring to FIG. 6, as a result of the measurement, when compared with the CON (normal) group, the length of the large intestine decreased in all groups administered with DSS. When compared with the DSS-alone treatment group, the decrease in the length of the large intestine was suppressed in the treatment group of the medium concentration (100 mg/kg) of the sesame oil meal extract.

### 4.3 Histological examination of large intestine

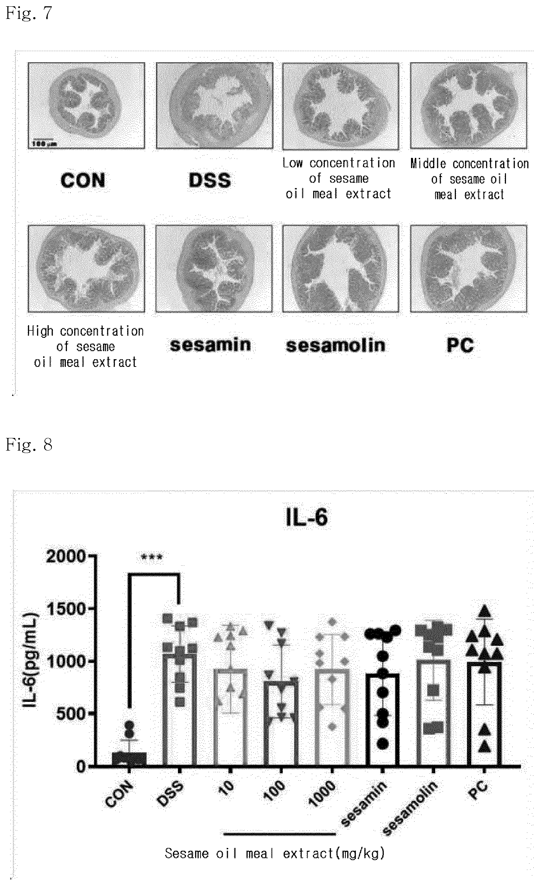

In order to determine whether the sesame oil meal extract has anti-colitis effect, mice were autopsied on the 8th day of the experiment, tissue of the large intestine was removed, and tissue slide sections were made and stained with H&E. It is known that the administration of DSS increases the large intestine lesions such as thickening of mucous membrane tissue, redness, an increase in inflammatory cells, and ulcers caused by tissue necrosis in the large intestine. Referring to FIG. 7, as a result of the measurement, when compared with the DSS-alone treatment group, thickening of mucous membrane tissues, redness, an increase in inflammatory cells, and ulcers due to tissue necrosis in the treatment group of the medium concentration (100 mg/kg) of the sesame oil meal extract, the degree of lesions of the large intestine tended to decrease.

### 4.4 Measurement of pro-inflammatory cytokines (IL-6, IL-1β, IFN-γ, TNF-α)

### 4.4.1 Measurement of IL-6

In order to determine whether the sesame oil meal extract has anti-colitis efficacy, mice were autopsied on the 8th day of the experiment to dissolve the large intestine tissue, and the concentration of pro-inflammatory cytokine (IL-6) in the tissue was measured. IL-6 is mainly produced by Th2 cells, is responsible for humoral immunity, and is known to cause inflammation when excessively secreted. Referring to FIG. 8, as a result of the measurement, when compared with the CON (normal) group, all groups administered with DSS showed an increase in IL-6. When compared with the DSS-alone treatment group, the concentration of IL-6 decreased in the treatment group of the medium concentration (100 mg/kg) of the sesame oil meal extract.

### 4.4.2 Measurement of IL-1β

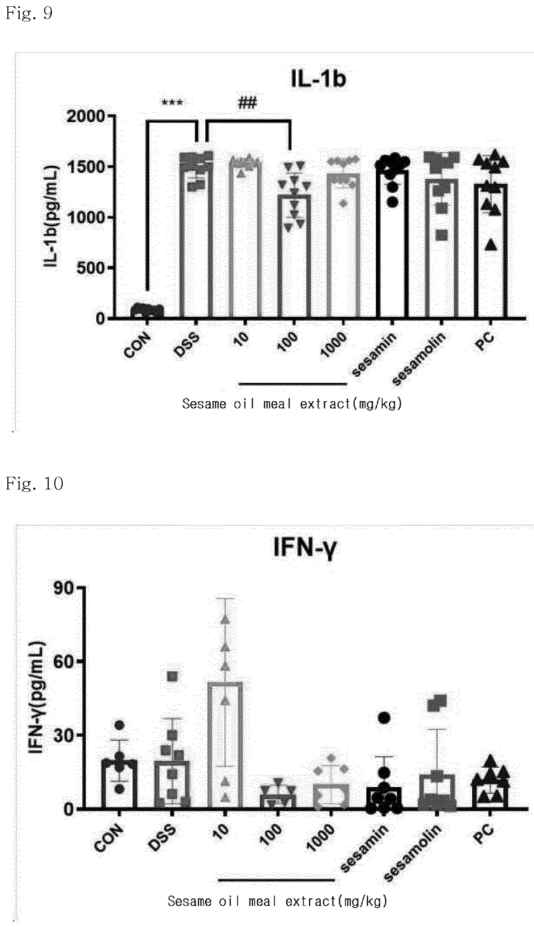

In order to determine whether the sesame oil meal extract has anti-colitis efficacy, mice were autopsied on the 8th day of the experiment to dissolve the large intestine tissue, and the concentration of pro-inflammatory cytokine (IL -1β) in the tissue was measured. IL-1β is known to be involved in cellular adaptive immunity and increases when colitis is induced. Referring to FIG. 9, as a result of the measurement, when compared with the CON (normal) group, all groups administered with DSS showed an increase in IL -1β. Compared with the DSS-alone treatment group, the concentration of IL-1β was significantly decreased in the treatment group of the medium concentration (100 mg/kg) of the sesame oil meal extract. Based on the results of this experiment, it can be seen that the sesame oil meal extract has anti-colitis efficacy by inhibiting the increase of IL-1β, which is responsible for cellular adaptive immunity.

### 4.4.3 Measurement of IFN-γ

In order to determine whether sesame oil meal extract has anti-colitis efficacy, mice were autopsied on the 8th day of the experiment to dissolve the large intestine tissue, and the concentration of pro-inflammatory cytokine (IFN-γ) in the tissue was measured. IFN-γ is mainly produced by Th1 cells, is responsible for cellular immunity, and is known to cause inflammation when excessively secreted. Referring to FIG. 10, as a result of the measurement, it can be seen that IFN-γ was not increased even in the DSS-administered group, so IFN-γ secretion was not induced in the DSS-administered mouse model.

### 4.4.4 Measurement of TNF-α

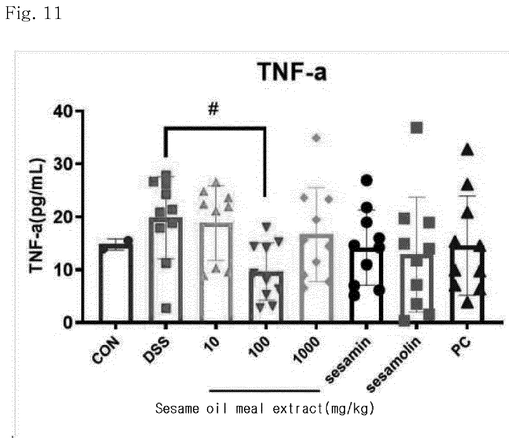

In order to determine whether the sesame oil meal extract has anti-colitis efficacy, mice were autopsied on the 8th day of the experiment to dissolve the large intestine tissue, and the concentration of pro-inflammatory cytokine (TNF-α) in the tissue was measured. TNF-α is mainly produced in Th1 cells, is responsible for cellular immunity, and is known to induce inflammation when excessively secreted. Referring to FIG. 11, as a result of the measurement, when compared with the CON (normal) group, all groups administered with DSS showed an increase in TNF-α. Compared with the DSS-alone treatment group, the concentration of TNF-α was significantly decreased in the treatment group of the medium concentration (100 mg/kg) of the sesame oil meal extract. Based on the results of this experiment, it can be seen that the sesame oil meal extract has anti-colitis efficacy by regulating the secretion of TNF-α of Th1, which is responsible for humoral immunity.

## Claims

1. A method for producing a sesame oil meal extract, the method comprising the steps of:
producing a sesame oil meal mixture by adding a solvent to sesame oil meal;
ultrasonically extracting the sesame oil meal mixture;
filtering and concentrating the ultrasonically extracted extract; and
drying the concentrated concentrate,
wherein the solvent is water,
wherein the sesame oil meal mixture is obtained by mixing the sesame oil meal to the solvent in a weight ratio (w/v) of 1:30,
wherein in the ultrasonic extraction step, an ultrasonic treatment is performed under conditions of a frequency of 10 to 50 kHz, a temperature of 20°C to 30°C, 3 to 5 hours, and an amplitude of 70 to 90%,
wherein in the concentration step, after the filtration using a housing filter with a filter size between 100 µm and 50 µm, the extract is concentrated under conditions of a temperature of 50°C to 60°C, a stirring of 15 to 25 RPM, a vacuum degree of -1200 to -930 Pascal (-9 to -7 torr), and an evaporation rate of 170 to 190 L/hr, and
wherein in the drying step, the concentrated concentrate is lyophilized.

2. The method of claim 1, wherein the ultrasonic extraction step is repeated 1 to 5 times.

3. The method of claim 1, wherein in the drying step, the concentrate is frozen under conditions of -50°C to -40°C and 5 to 7 hours, and is dried under the conditions of 25°C to 35°C and 48 to 52 hours.

4. A food composition for use in preventing or improving inflammatory diseases, the food composition comprising a sesame oil meal extract produced by the method of any one of claims 1 to 3 as an active ingredient,
wherein the inflammatory disease is colitis accompanied with weight loss, stool abnormalities, rectal bleeding or a reduction in colon length.

5. The food composition for use according to claim 4, wherein the food is a health functional food.

6. Sesame oil meal extract produced by the method according to any one of claims 1 to 3 for use in preventing or treating an inflammatory diseases
wherein the inflammatory disease is colitis accompanied with weight loss, stool abnormalities, rectal bleeding or a reduction in colon length.

7. The sesame oil meal extract for use of claim 6, wherein the extract inhibits body weight loss, stool abnormalities, rectal bleeding, and a reduction in colon length, and reduces the expression of IL-6, IL-1β, and TNF-α.

## Patentansprüche

1. Verfahren zur Herstellung eines Sesamölmehlextrakts, wobei das Verfahren die Schritte umfasst:
Herstellen eines Sesamölmehlgemischs durch Zugeben eines Lösungsmittels zu Sesamölmehl;
Ultraschallextrahieren des Sesamölmehlgemischs;
Filtrieren und Konzentrieren des ultraschallextrahierten Extrakts; und
Trocknen des konzentrierten Konzentrats,
wobei das Lösungsmittel Wasser ist,
wobei das Sesamölmehlgemisch durch Mischen des Sesamölmehls mit dem Lösungsmittel in einem Gewichtsverhältnis (w/v) von 1:30 erhalten wird,
wobei bei dem Ultraschallextraktionsschritt eine Ultraschallbehandlung unter Bedingungen einer Frequenz von 10 bis 50 kHz, einer Temperatur von 20 °C bis 30 °C, 3 bis 5 Stunden und einer Amplitude von 70 bis 90 % durchgeführt wird,
wobei bei dem Konzentrationsschritt nach der Filtration unter Verwendung eines Gehäusefilters mit einer Filtergröße zwischen 100 µm und 50 µm der Extrakt unter Bedingungen einer Temperatur von 50 °C bis 60 °C, Rühren mit 15 bis 25 U/min, eines Vakuumniveaus von -1200 bis -930 Pascal (-9 bis -7 Torr) und einer Verdampfungsrate von 170 bis 190 l/h konzentriert wird, und
wobei bei dem Trocknungsschritt das konzentrierte Konzentrat lyophilisiert wird.

2. Verfahren nach Anspruch 1, wobei der Ultraschallextraktionsschritt 1 bis 5 Mal wiederholt wird.

3. Verfahren nach Anspruch 1, wobei bei dem Trocknungsschritt das Konzentrat unter Bedingungen von -50 °C bis -40 °C und 5 bis 7 Stunden gefroren wird und unter den Bedingungen von 25 °C bis 35 °C und 48 bis 52 Stunden getrocknet wird.

4. Lebensmittelzusammensetzung zur Verwendung bei der Vorbeugung oder Verbesserung von entzündlichen Erkrankungen, wobei die Lebensmittelzusammensetzung einen Sesamölmehlextrakt, hergestellt durch das Verfahren nach einem der Ansprüche 1 bis 3, als einen Wirkstoff umfasst,
wobei die entzündliche Erkrankung Colitis, einhergehend mit Gewichtsverlust, Stuhlanomalien, Rektalblutung oder einer Verringerung der Colonlänge, ist.

5. Lebensmittelzusammensetzung zur Verwendung nach Anspruch 4, wobei das Lebensmittel ein Gesundheitsfunktionslebensmittel ist.

6. Sesamölmehlextrakt, hergestellt durch das Verfahren nach einem der Ansprüche 1 bis 3, zur Verwendung bei der Vorbeugung oder Behandlung einer entzündlichen Erkrankung,
wobei die entzündliche Erkrankung Colitis, einhergehend mit Gewichtsverlust, Stuhlanomalien, Rektalblutung oder einer Verringerung der Colonlänge, ist.

7. Sesamölmehlextrakt zur Verwendung nach Anspruch 6, wobei der Extrakt Körpergewichtsverlust, Stuhlanomalien, Rektalblutung und eine Verringerung der Colonlänge hemmt und die Expression von IL-6, IL-1β und TNF-α verringert.

## Revendications

1. Procédé de production d'un extrait de farine d'huile de sésame, le procédé comprenant les étapes de :
production d'un mélange de farine d'huile de sésame par ajout d'un solvant à la farine d'huile de sésame ;
extraction par ultrasons du mélange de farine d'huile de sésame ;
filtration et concentration de l'extrait obtenu par extraction par ultrasons ; et
séchage du concentré obtenu par concentration,
dans lequel le solvant est l'eau,
dans lequel le mélange de farine d'huile de sésame est obtenu en mélangeant la farine d'huile de sésame au solvant dans un rapport en poids (p/v) de 1:30,
dans lequel, dans l'étape d'extraction par ultrasons, un traitement par ultrasons est réalisé dans des conditions d'une fréquence de 10 à 50 kHz, d'une température de 20°C à 30°C, pendant 3 à 5 heures et d'une amplitude de 70 à 90%,
dans lequel, dans l'étape de concentration, après la filtration en utilisant un filtre à boîtier ayant une taille de filtre comprise entre 100 µm et 50 µm, l'extrait est concentré dans des conditions d'une température de 50°C à 60°C, d'une agitation de 15 à 25 tr/min, d'un degré de vide de -1200 à -930 pascals (-9 à -7 torr) et d'un taux d'évaporation de 170 à 190 L/h, et
dans lequel, dans l'étape de séchage, le concentré obtenu par concentration est lyophilisé.

2. Procédé de la revendication 1, dans lequel l'étape d'extraction par ultrasons est répétée 1 à 5 fois.

3. Procédé de la revendication 1, dans lequel, dans l'étape de séchage, le concentré est congelé dans des conditions de -50°C à -40°C et pendant 5 à 7 heures, et est séché dans des conditions de 25°C à 35°C et pendant 48 à 52 heures.

4. Composition alimentaire pour une utilisation dans la prévention ou l'atténuation de maladies inflammatoires, la composition alimentaire comprenant un extrait de farine d'huile de sésame produit par le procédé de l'une quelconque des revendications 1 à 3 comme principe actif,
dans laquelle la maladie inflammatoire est une colite accompagnée d'une perte de poids, d'anomalies de selles, de saignements rectaux ou d'une réduction de la longueur du côlon.

5. Composition alimentaire pour une utilisation selon la revendication 4, dans laquelle l'aliment est un aliment fonctionnel de santé.

6. Extrait de farine d'huile de sésame produit par le procédé selon l'une quelconque des revendications 1 à 3 pour une utilisation dans la prévention ou le traitement de maladies inflammatoires
dans lequel la maladie inflammatoire est une colite accompagnée d'une perte de poids, d'anomalies de selles, de saignements rectaux ou d'une réduction de la longueur du côlon.

7. Extrait de farine d'huile de sésame pour une utilisation selon la revendication 6, dans lequel l'extrait inhibe la perte de poids, les anomalies de selles, les saignements rectaux et la réduction de la longueur du côlon, et réduit l'expression de IL-6, IL-1β et TNF-α.
